# EUROPEAN PATENT SPECIFICATION

(11) **EP 4 440 654 B1**
(45) Date of publication and mention of the grant of the patent: **08.04.2026**
(21) Application number: 22871173.5
(22) Date of filing: 05.12.2022
(51) Int. Cl.: A61M 5/158

(54) **MEDICAL SAFETY NEEDLE ASSEMBLY**
MEDIZINISCHE SICHERHEITSNADELANORDNUNG
ENSEMBLE AIGUILLE DE SÉCURITÉ MÉDICALE

(30) Priority: 03.12.2021 FR 2112968
(43) Date of publication of application: 09.10.2024
(73) Proprietor: Design 33 Inc., Cheyenne, WY 82001 (US)
(72) Inventor: FORBER, Simon, John, 86240 Ligugé (FR)
(74) Representative: Ter Meer Steinmeister & Partner
(86) International application number: PCT/IB2022/000678
(87) International publication number: WO 2023/099955

(56) References cited:
- FR-A1- 2 916 979
- US-A1- 2008 269 695

## Description

The present invention relates to a medical safety needle assembly.

Implantable vascular access systems are used extensively in the medical field to facilitate the performance of repeated injections of therapeutic treatments inside the body of a patient The vascular access system is generally comprised of a vascular access port that is attached to a catheter. The vascular access port generally consists of a housing with an elastomeric septum exposed on the upper surface. Beneath the elastomeric septum is a reservoir that can be accessed by the tip of a needle when it passes the elastomeric septum. The vascular access port has a tubular elongated member extending from the reservoir that allows attachment of the catheter.

The vascular access system is implanted into the body of a patient. The distal tip of the catheter is disposed at a predetermined location where the therapeutic agent is delivered, generally the entrance of the right atrium just beyond the superior vena cava. Once the vascular access system is implanted, the care giver can insert the tip of the needle through the skin, the elastomeric septum and into the reservoir space to deliver the treatment. In order to minimize the height of the needle extending from the skin, the needles are bent approximately 90° so that they extend minimally above the skin of the patient

The tip of the needle has a bend close to the pnnciple bevel of the needle. This bend is such that once formed the pnnciple bevel is approximately parallel to the axis of the unbent portion of the cannula The generally accepted terminology for this type of needle tip is a "Huber" tip When a needle of this conception is forced through the elastomeric septum, It creates just a slit in the septum When the needle is removed, the slit surfaces return in contact and under the radial compression of the septum and the Intrinsic charactenstics of the elastomeric material, a seal is maintained If a regular hypodermic needle was inserted into the elastomeric septum there is an elevated risk that the needle may core particles from the septum. This could potentially impair the septum and/or liberate the particles that could obstruct the catheter or be released into the vasculature of the patient

As with any needle type device, there exists the risk of inadvertent needle sticks, that generally occur when the needle is removed from the patient prior to disposal of the needle This risk is particularly significant in the case of Huber needles being removed from a vascular access system This is because the force to remove a Huber needle from a port is high due the fact that the needle is being compressed radially by the elastomenc septum of the vascular access port. Also the care giver generally needs to stabilize the vascular access port with the fingers of the opposite hand at a location close to the exit site of the needle.

To address the problem of inadvertent needle sticks, various safety devices have been designed. One safety device is described in US 5,765,694 that discloses a needle base disposed over a segment of the needle at its proximal end, compnsing two generally flat wings made of a flexible plastic material, with one hinge connecting each of the wings to the needle base Upon removal of the needle from the patient, the wings flex against the moveable members that keeps them adjacent to the needle until the needle is completely removed from the skin of the patient after which the wings surround the needle to prevent the tip from making contact outside the needle base.

Many advancements have been made in needle devices for use with vascular access ports to reduce the risk of needle sticks Additionally there is another potential problem. When the needle is inserted through the septum, it occupies a volume corresponding to the volume of the needle and liquid therein that extends below the elastomeric septum When the needle is removed from the vascular access port, this volume of the needle and liquid within is removed without replacement, causing an equivalent volume of venous blood to draw into the distal end of the catheter. This infiltration of blood is suspected to cause or potentially contribute to thrombus formation at the tip of the catheter, which could block the flow of fluid through the catheter The risk is dependent on the inner diameter of the catheter as the equivalent volume of blood drawn back equates to a short column of blood in a large catheter, whereas in a small diameter catheter, the equivalent catheter. This early removal is costly, invasive, time consuming and otherwise completely undesirable.

One solution to this problem has been to implement a positive flushing technique. This technique consists of the medical practitioner inserting a syringe to the hub of the "Huber" needle and injecting heparinized saline into the port while simultaneously withdrawing the needle. The effect being, that the injected volume of liquid compensates for the volume of the needle that is being removed from the reservoir and the port. However this technique is quite cumbersome and complicated and in many cases cannot be done by a single care giver as many safety needles require two hands to operate. One such device that allows for positive flushing by one care giver is described in US 7,927,317 that discloses a needle with a two part foldable needle cover. When the needle is in the patient, the two part cover is folded down. To remove the needle the care giver lifts the cover to the vertical position, then places the thumb on the top of the fixed part of the cover. The care giver then places the forefinger and middle finger on the flanges extending on the moveable part of the cover. In this position, the care giver pulls up on the moveable part of the cover that in turn pulls the needle up and into the fixed part of the cover. At the end of the travel, the moveable portion of the cover locks to the fixed part of the cover preventing the needle from moving downwards. Because only one hand is required for this operation it frees up the second hand to slowly inject liquid through the syringe as the first hand removes the needle.

This technique is effective, however this device is cumbersome and has several significant drawbacks. Firstly the size of the cover and mechanism is very large. The larger the device is above the skin, the higher the risk that the needle might be inadvertently hit and become dislodged from the patient. Also the fact that the device extends significantly above the skin of the patient is cosmetically undesirable. Additionally, during the removal of the device, the limited size of the device's contact pad and the force required to remove the needle can be very uncomfortable.

FR 2 916 979 A1 discloses an injection device with an extraction mechanism for a Huber needle to extract and protect the needle. The mechanism comprises a pushing member and a sleeve to extract the needle by one hand.

US 2008/269695 A1 discloses an injection device with an extraction mechanism comprising a base in a pushing member. The base is pulled by the caregiver to extract the needle by one hand.

Accordingly, the object of the present invention is to provide a medical needle assembly that is easy to remove and that provides the possibility of performing a positive flushing during needle removal by one single person

The object of the invention is achieved with a medical safety needle assembly comprising a medical needle having a distal and a proximal end and a stem extending there between The needle has a tip at its distal end, and the proximal end of the needle is connected to a tubing with a connection hub for connecting the tubing to a syringe, wherein the connection is able to resist tensile loads. The assembly further comprises a base plate The needle extends slidably through the base plate and has an inserted position with the needle tip extending in distal direction from the base plate and a retracted position with the needle tip being in a position inside a cavity of the base plate.

The medical needle assembly of the invention is characterized in that the needle comprises a first attachment element at its proximal side of the base plate, and in that the connection hub comprises a second attachment element being able to engage with the first attachment element in order to pull the needle through the base plate into its retracted position

The first attachment element of the needle may in particular be an eye, and the second attachment element of the connection hub a hook that may engage with the eye of the needle, so that the needle may be pulled back from its inserted position into its retracted position. However, the first and second attachment elements may be any kind of element that may engage with each other in a way that a force applied on the connection hub is transferred to the needle to bring it from the inserted into the retracted position. In particular, any form-locked or any force-locked connection may be used

When the needle is in its inserted position, the distal side of the base plate is in contact with a patient's skin, while the needle tip may for example be inserted into a vascular access system. A liquid may be injected into the vascular access system when a synnge or another suitable device like an infusion pump is connected to the connection hub of the assembly

A tubing, in particular a flexible tube, is attached between the proximal end of the needle and the connector hub that creates a continuous fluid path from the tip of the needle to the connector hub.

The connection hub may be a rigid part with a central lumen that extends through the length of the part.

The connection possibility provided by the connection hub shall be able to resist tensile loads. For this purpose, at one end of the lumen of the connector, the internal and external form and dimensions may for example conform to the ISO standard for Luer connectors (ISO 80369-7) In such case, a standard syringe with a corresponding Luer connector may be connected with the connection hub, and the connections resists tensile loads in the direction of the lumen of the connector

The opposite end is dimensioned to allow for the bonding of the tubing

The connector hub may preferably have one or more radial protrusions from the body of the part, the protrusions aide in applying the torque necessary to attach the connector hub to another connector, preferably a Luer connector

The second attachment element of the connector hub, e.g a hook, shall be able to engage with the first attachment element of the needle, e.g. an eye, so that both parts may be connected by a caregiver. For removal of the needle, a syringe may be connected to the connection hub, and the second attachment element may be attached to the first attachment element together with the syringe The caregiver may then hold with one hand the base plate down on the skin of the patient and may pull the syringe upwards with the other hand, thus removing the needle from the patient. At the same time, the caregiver may apply a light pressure on the syringe so that some liquid is inserted through the needle into the vascular access port or other device. The removal of the needle in this way does not create a backflow of blood into an implanted device but actively flushes it so that no blood is pulled into the thin vascular access system tube.

Preferably, a handle may be attached to the needle stem at its proximal side of the base plate Such a handle may help the caregiver to introduce the needle into a patient's body, and such handle may in particular comprise the first attachment element, such as an opening forming an eye for a hook.

In a preferred embodiment of the present invention, the needle stem may have a 80°-90° bend at its proximal part above the base plate. The bend may be positioned such that it is close to the base plate when the needle is in its inserted position so that the assembly has a low profile and does not extend significantly above the skin of the patient.

In order to ensure straight insertion and removal of the needle, the handle may advantageously be attached close to the bend of the needle.

The handle may be movable between a position parallel to the base plate and a position vertical to the base plate, so that pressure in the distal direction of the needle may be applied for inserting the needle, and that tension close to the needle shaft may be applied for removing the needle by pulling on a synnge.

In an embodiment of the invention, the handle may consist of two parts that may move independently, e.g in the form of two wings. The two parts of the handle may extend into opposite directions in their position parallel to the base plate Both parts may also form a single handle when they are in the vertical position. Preferably, they may engage with each other in the vertical position, e g by means of suitable connection elements like e g. interconnecting and releasable clips.

The assembly comprises preferably safety means that prevent the needle tip from leaving the cavity inside of the base plate. Such means are well known in the state of the art and not subject of this invention

Various embodiments of the invention are described in greater detail in the following, using the attached figures:
**Figure 1** is an isometric view of one embodiment of the present invention in the inserted needle position;
**Figure 2** is an isometric view of the base plate and the needle of the embodiment of figure 1 after insertion of the needle with the tubing and the connecting hub removed;
**Figure 3** shows the embodiment of figure 2 in the inserted needle position with the handle folded down to achieve a low profile,
**Figure 4** is an isometric view of the base plate and needle of figure 3 in the retracted needle position;
**Figure 5** is an isometric view the embodiment of the invention of the previous figures in the inserted needle position, prepared for needle removal with positive flushing:
**Figure 6** is an isometric view of the assembly of figure 5 in the retracted needle position,

It will be further understood that the terms "compnses" and/or "comprising" when used in this specification, specify the presence of stated features, steps, operations, elements and/or components, but do not preclude the presence or addition of one or more other features, steps, operations, elements, components, and/or groups thereof

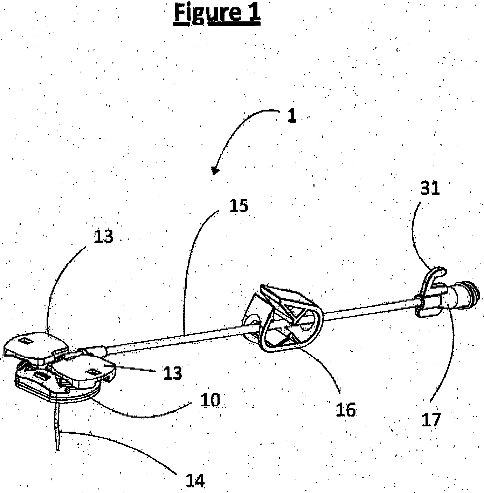

**Figure 1** is an isometric view of one embodiment of the medical safety needle assembly **1** in accordance with the present invention in the inserted needle position. The assembly comprises a base plate **10,** a handle **13** comprising two parts, a needle **14,** a tubing **15** and a connecting hub **17** In addition, a clamp **16** may be positioned on the tubing **16.** The handle **13** comprises two parts that are movable between a position parallel to the base plate **10** shown in the figure and a position vertical to the base plate. The two parts of the handle **13** have each an opening **32** that form an eye in the vertical position of the handle parts, so that the hook **31** of the connection hub **17** may engage therein

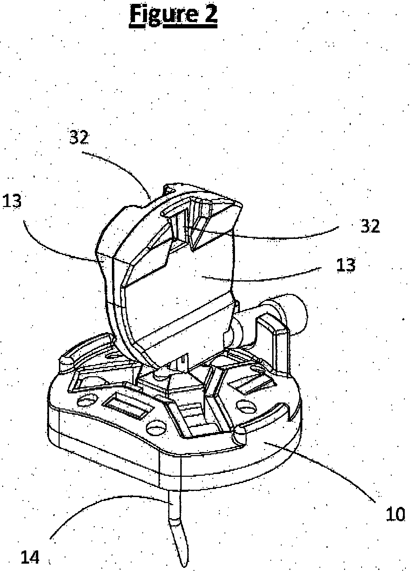

**Figure 2** is a closer isometric view of the base plate **10** with the needle **14** in the inserted needle position. To insert the needle **14** in the patient, the two wing-shaped parts of the handle **13** are folded up in a position essentially vertical to the base plate **10** allowing the care giver the stability to push the needle down through the skin and into the reservoir of a vascular access system

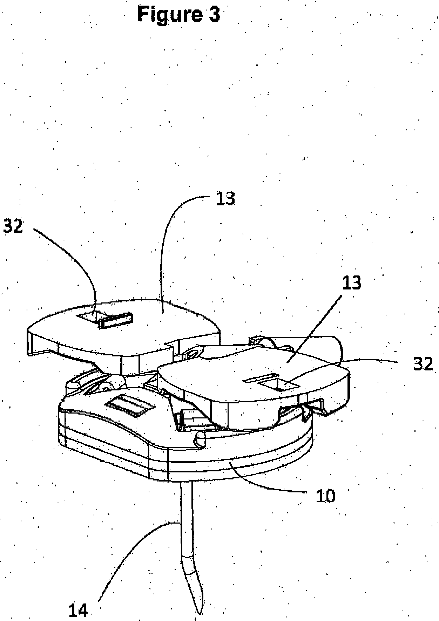

After insertion of the needle, the wings may be folded down to achieve a low profile, as is shown in **Figure 3****.**

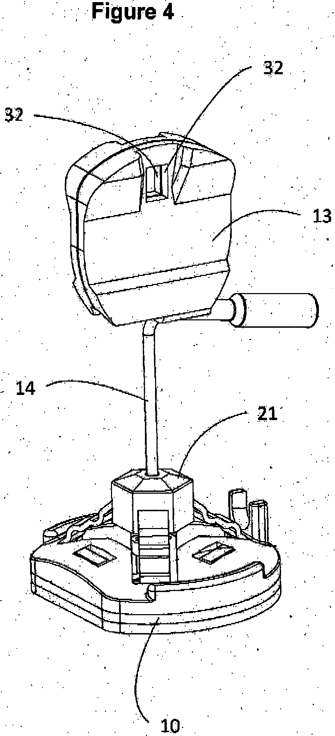

**Figure 4** is an isometric view of the elements shown in figure 3, but in the retracted position of the needle To achieve this position, the care giver uses two fingers of one hand to push on the cover of the base plate **10** to maintain the base plate in contact with the patient's skin. The two wing-shaped parts of the handle **13** are folded up in a position essentially vertical to the base plate **10,** and the care giver pulls up on the handle **13** vertically, using two fingers of the opposite hand until the needle tip is enclosed in a cavity within the base plate A safety mechanism prevents the needle from leaving the cavity within the base plate in either direction, further upwards or further downwards. This mechanism is not part of the present invention, and suitable solutions are well-known in the state of the art

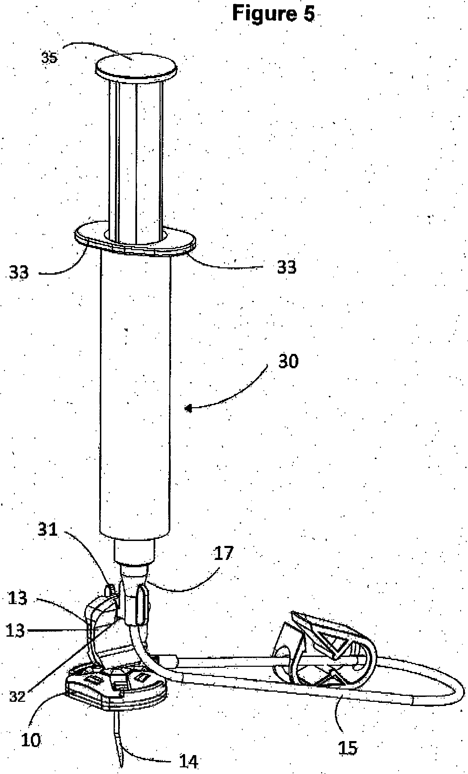

**Figure 5** is a an isometric view of a medical safety needle assembly in accordance with the previous figures in the inserted needle position to allow for positive flushing during needle removal. In the shown configuration the connector hub **17** is first attached to a syringe **30** containing fluid, generally heparinized physiological saline. The care giver folds two movable parts of the handle **13** into their position vertical to the base plate **10** so that the two openings **32** form an eye

The caregiver then inserts the hook **31** on the connector hub **17** through the eye of the handle **13** The care giver uses two fingers of one hand to maintain the base plate assembly **10** in contact with the patient's skin. With the opposite hand, the care giver can hold the syringe **30** in the standard manner, the thumb on the piston **35** and the index and middle fingers beneath the syringes flanges **33** The care giver can then pull up on the synnge **30** with the two fingers beneath the flanges **33** which will in turn pull up on the handle **13** and needle **14.** At the same time slight pressure can be applied to the piston **35** during removal of the needle **14,** achieving positive flushing during removal. It is obvious that depending on the care giver they can use this positive flushing technique or decide not use positive flushing and just pull the needle up on the handle **13** directly with the opposite hand.

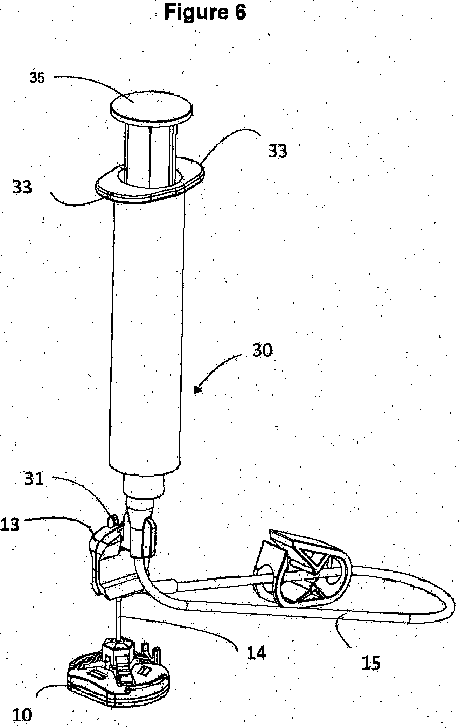

**Figure 6** is an isometric corresponding to figure 4 but in the removed needle position, wherein the needle tip is enclosed in a cavity within the base plate **10** after retraction of the needle **14.**

## Claims

1. Medical safety needle assembly (1) comprising
a medical needle (14) having a distal and a proximal end and a stem extending there between,
the needle having a tip at its distal end,
the proximal end of the needle being connected to a tubing (15) with a connection hub (17) for connecting the tubing (15) to a syringe, the connection being able to resist tensile loads,
and a base plate (10)
the needle (14) extending slidably through the base plate (10) and having an inserted position with the needle tip extending in distal direction from the base plate (10) and a retracted position with the needle tip being in a position inside a cavity of the base plate,
**characterized in that**
the needle comprises a first attachment element (32) at its proximal side of the base plate (10),
and the connection hub (17) comprises a second attachment element (31) being able to engage with the first attachment element (32) in order to pull the needle (14) through the base (10), plate into its retracted position.

2. Safety needle assembly according to claim 1, **characterized in that** the first attachment element is an eye (32).

3. Safety needle assembly according to claim 1 or 2, **characterized in that** the second attachment element is a hook (31).

4. Safety needle assembly according to any one of claims 1 to 3, **characterized, in that** a handle (13) is attached to the needle stem at its proximal side of the base plate (10).

5. Safety needle assembly according to claim 4, **characterized in that** the handle (13) comprises the first attachment element, in particular an opening (32) as an eye.

6. Safety needle assembly according to any one of claims 1 to 5, **characterized in that** the needle stem has a 80°-90° bend at its proximal part of the base plate.

7. Safety needle assembly according to any one of claims 4 to 6, **characterized in that** the handle (13) is attached close to the bend.

8. Safety needle assembly according to any one of claims 4 to 7, **characterized in that** the handle (13) or parts thereof are movable between a position parallel to the base plate (10) and a position vertical to the base plate.

9. Safety needle assembly according to any one of claims 4 to 8, **characterized in that** the handle (13) consists of two parts that may move independently.

10. Safety needle assembly according to claim 9, **characterized in that** the two parts of the handle (13) extend into opposite directions in their position parallel to the base plate (10).

11. Safety needle assembly according to claim 9 or 10, **characterized in that** both parts form a single handle when they are in the vertical position.

12. Safety needle assembly according to any one of claims 9 to 11, **characterized in that** both parts of the handle engage with each other in the vertical position.

## Patentansprüche

1. Medizinische Sicherheitsnadelanordnung (1), die Folgendes umfasst:
eine medizinische Nadel (14), die ein distales und ein proximales Ende und einen Schaft, der sich dazwischen erstreckt, aufweist,
wobei die Nadel an ihrem distalen Ende eine Spitze aufweist,
wobei das proximale Ende der Nadel mit einem Schlauch (15) mit einer Verbindungsmuffe (17) zum Verbinden des Schlauchs (15) mit einer Spritze verbunden ist, wobei die Verbindung Zugbelastungen widerstehen kann,
und eine Grundplatte (10),
wobei sich die Nadel (14) gleitfähig durch die Grundplatte (10) erstreckt und eine eingesetzte Position, in der sich die Nadelspitze in der distalen Richtung von der Grundplatte (10) erstreckt, und eine eingezogene Position, in der sich die Nadel an einer Position im Inneren eines Hohlraums der Grundplatte befindet, aufweist,
**dadurch gekennzeichnet, dass**
die Nadel ein erstes Befestigungselement (32) auf ihrer proximalen Seite der Grundplatte (10) aufweist
und die Verbindungsmuffe (17) ein zweites Befestigungselement (31) aufweist, das mit dem ersten Befestigungselement (32) in Eingriff gelangen kann, um die Nadel (14) durch die Grundplatte (10) an ihre eingezogene Position zu ziehen.

2. Sicherheitsnadelanordnung nach Anspruch 1, **dadurch gekennzeichnet, dass** das erste Befestigungselement eine Öse (32) ist.

3. Sicherheitsnadelanordnung nach Anspruch 1 oder 2, **dadurch gekennzeichnet, dass** das zweite Befestigungselement ein Haken (31) ist.

4. Sicherheitsnadelanordnung nach einem der Ansprüche 1 bis 3, **dadurch gekennzeichnet, dass** ein Griff (13) auf seiner proximalen Seite der Grundplatte (10) am Nadelschaft befestigt ist.

5. Sicherheitsnadelanordnung nach Anspruch 4, **dadurch gekennzeichnet, dass** der Griff (13) das erste Befestigungselement, insbesondere eine Öffnung (32) als eine Öse, aufweist.

6. Sicherheitsnadelanordnung nach einem der Ansprüche 1 bis 5, **dadurch gekennzeichnet, dass** der Nadelschaft eine Krümmung im Bereich von 80°-90° an seinem proximalen Abschnitt der Grundplatte aufweist.

7. Sicherheitsnadelanordnung nach einem der Ansprüche 4 bis 6, **dadurch gekennzeichnet, dass** der Griff (13) nahe an der Krümmung befestigt ist.

8. Sicherheitsnadelanordnung nach einem der Ansprüche 4 bis 7, **dadurch gekennzeichnet**, das der Griff (13) oder Abschnitte davon zwischen einer Position parallel zur Grundplatte (10) und einer Position vertikal zur Grundplatte beweglich sind.

9. Sicherheitsnadelanordnung nach einem der Ansprüche 4 bis 8, **dadurch gekennzeichnet, dass** der Griff (13) aus zwei Abschnitten besteht, die sich unabhängig bewegen können.

10. Sicherheitsnadelanordnung nach Anspruch 9, **dadurch gekennzeichnet, dass** sich die zwei Abschnitte des Griffs (13) in ihrer Position parallel zur Grundplatte (10) in entgegengesetzten Richtungen erstrecken.

11. Sicherheitsnadelanordnung nach Anspruch 9 oder 10, **dadurch gekennzeichnet, dass** beide Abschnitte einen einzigen Griff bilden, wenn sie sich in der vertikalen Position befinden.

12. Sicherheitsnadelanordnung nach einem der Ansprüche 9 bis 11, **dadurch gekennzeichnet, dass** sich beide Abschnitte des Griffs in der vertikalen Position miteinander in Eingriff befinden.

## Revendications

1. Ensemble d'aiguille de sécurité médicale (1) comportant :
une aiguille médicale (14) ayant une extrémité distale et une extrémité proximale et une tige s'étendant entre celles-ci,
l'aiguille ayant une pointe à son extrémité distale,
l'extrémité proximale de l'aiguille étant raccordée à un tube (15) avec une embase de raccordement (17) pour raccorder le tube (15) à une seringue, le raccordement étant capable de résister à des charges de traction,
et une plaque de base (10)
l'aiguille (14) s'étendant de manière coulissante à travers la plaque de base (10) et ayant une position insérée avec la pointe d'aiguille s'étendant dans une direction distale à partir de la plaque de base (10), et une position rétractée avec la pointe d'aiguille étant dans une position à l'intérieur d'une cavité de la plaque de base,
**caractérisé en ce que**
l'aiguille comporte un premier élément de fixation (32) sur son côté proximal de la plaque de base (10),
et l'embase de raccordement (17) comporte un second élément de fixation (31) étant capable de venir en prise avec le premier élément de fixation (32) afin de tirer l'aiguille (14) à travers la plaque de base (10) dans sa position rétractée.

2. Ensemble d'aiguille de sécurité selon la revendication 1. **caractérisé en ce que** le premier élément de fixation est un œillet (32).

3. Ensemble d'aiguille de sécurité selon la revendication 1 ou 2, **caractérisé en ce que** le second élément de fixation est un crochet (31).

4. Ensemble d'aiguille de sécurité selon l'une quelconque des revendications 1 à 3, **caractérisé en ce qu'**une poignée (13) est fixée à la tige d'aiguille sur son côté proximal de la plaque de base (10).

5. Ensemble d'aiguille de sécurité selon la revendication 4, **caractérisé en ce que** la poignée (13) comporte le premier élément de fixation, en particulier une ouverture (32), en tant qu'œillet.

6. Ensemble d'aiguille de sécurité selon l'une quelconque des revendications 1 à 5, **caractérisé en ce que** la tige d'aiguille a un coude de 80° à 90° sur sa partie proximale de la plaque de base.

7. Ensemble d'aiguille de sécurité selon l'une quelconque des revendications 4 à 6, **caractérisé en ce que** la poignée (13) est fixée près du coude.

8. Ensemble d'aiguille de sécurité selon l'une quelconque des revendications 4 à 7, **caractérisé en ce que** la poignée (13) ou des parties de celle-ci sont mobiles entre une position parallèle à la plaque de base (10) et une position verticale à la plaque de base.

9. Ensemble d'aiguille de sécurité selon l'une quelconque des revendications 4 à 8, **caractérisé en ce que** la poignée (13) est constituée de deux parties qui peuvent se déplacer indépendamment.

10. Ensemble d'aiguille de sécurité selon la revendication 9, **caractérisé en ce que** les deux parties de la poignée (13) s'étendent dans des directions opposées dans leur position parallèle à la plaque de base (10).

11. Ensemble d'aiguille de sécurité selon la revendication 9 ou 10, **caractérisé en ce que** les deux parties forment une seule poignée lorsqu'elles sont dans la position verticale.

12. Ensemble d'aiguille de sécurité selon l'une quelconque des revendications 9 à 11, **caractérisé en ce que** les deux parties de la poignée viennent en prise l'une avec l'autre dans la position verticale.
